# EUROPÄISCHE PATENTSCHRIFT

(11) **EP 1 430 922 B1**
(45) Veröffentlichungstag und Bekanntmachung des Hinweises auf die Patenterteilung: **17.05.2006**
(21) Anmeldenummer: 03025861.0
(22) Anmeldetag: 12.11.2003
(51) Int. Cl.: A61M 5/44

(54) **Vorrichtung zum Temperieren von in einem Schlauch geführter Flüssigkeit**
Device for maintaining fluid temperature in a tube
Dispositif pour maintenir la température d'un liquide dans une tube

(30) Priorität: 19.11.2002 DE 10254050
(43) Veröffentlichungstag der Anmeldung: 23.06.2004
(73) Patentinhaber: Transmed Medizintechnik GmbH & Co. KG, 33181 Bad Wünnenberg (DE)
(72) Erfinder: Bartscher, Rainer, 33181 Wünnenberg (DE); Pelster, Michael, 59581 Warstein-Hirschberg (DE)
(74) Vertreter: Valentin, Ekkehard

(56) Entgegenhaltungen:
- DE-A- 4 223 521
- DE-A- 19 740 177
- US-A- 3 096 426

## Beschreibung

Die Erfindung betrifft eine Vorrichtung zum Temperieren von in einem Schlauch geführter Flüssigkeit, insbesondere ein Blutprodukt oder eine Infusionslösung, umfassend einen Wickelkörper mit sich entlang des Umfangs seiner Mantelfläche erstreckenden, spiral- oder gewindeartigen, einen Führungsgang zur Aufnahme des aufgewickelten Schlauches bildenden Nuten.

Blutprodukte, wie Erythrozytenkonzentrat oder Vollblut, werden in der Regel mit üblichen Temperaturen von 2 bis 6°C kalt gelagert, um die Haltbarkeit zu erhöhen. Die zur Verwendung kommenden Infusionslösungen werden entweder in einem Kühlschrank bei 2 bis 6°C oder bei Raumtemperatur von etwa 20°C aufbewahrt. Diese einem Patienten intravenös über einen Venenzugang zugeführten Flüssigkeiten müssen aber so erwärmt werden, daß sie zum Zeitpunkt des Veneneintritts körperwarm (35 bis 37°C) sind. Bei Verletzungen mit hohem Blutverlust, hervorgerufen durch mehrere Körperöffnungen, kann es erforderlich sein, eine große Menge des Blutproduktes innerhalb einer sehr kurzen Zeit zu erwärmen. Bei einem Patienten mit einem Körpergewicht von 70 kg können bis zu 105 ml Erythrozytenkonzentrat pro Minute verabreicht werden, das mit Hilfe einer Erwärmvorrichtung von 4°C Anfangstemperatur auf Körpertemperatur temperiert werden muß.

Es ist üblich, in Kunststoffbeuteln abgefüllte Blutprodukte oder in Flaschen bzw. Kunststoffbeuteln bevorratete Infusionslösungen an einen Infusionsständer in etwa 1,5 bis 2 m Höhe aufzuhängen. Von dort strömt das Blutprodukt bzw. die Infusionslösung durch einen sogenannten Infusionsschlauch zum Venenzugang.

Zur Erwärmung des Blutes bzw. einer Infusionslösung auf seinem Weg vom Bevorratungsbehältnis bis zum Venenzugang ist durch die DE 197 40 177 A1 eine gattungsgemäße Vorrichtung bekanntgeworden, bei der der Schlauchwickler als zylindrischer Körper mit einem spiralförmigen Umfangsgewinde als Führungsgänge für den Schlauch ausgebildet ist. An dem zylindrischen Körper ist ein Stecker für die elektrische Energiezufuhr zu einer Heizvorrichtung sowie ein Bedienfeld mit Regel- und Anzeigevorrichtungen für die Steuerung der Heizvorrichtung vorgesehen.

Der zylindrische Wickelkörper wird mit seiner Rückseite in waagerechter Position an einem Infusionsständer befestigt und der Schlauch von Hand von hinten nach vorne in die Nut des Führungsgangs eingelegt. Damit der Schlauch im eingelegten Zustand innerhalb der Nut fixiert wird, ist diese mit einer Verengung in radialer Richtung von innen nach außen versehen. Um den Schlauch in seine ohne äußere Krafteinwirkung lagesichere Position in der Nut gelangen kann, muß er beim Einwickeln unter ständiger gerichteter Krafteinwirkung vollständig in die Nut bis zum Nutgrund hineingezogen werden. Dieses sorgfältige Einlegen in die Nut bereitet erhebliche Schwierigkeiten. Denn eine vor dem Wickelkörper stehende und ihre Blickposition nicht verändernde Person hat keine freie Sicht auf die gesamte Oberfläche des zylindrischen Wickelkörpers. Das Finden der Nut sowie das korrekte Einlegen des Schlauchs bis in den Nutgrund ist daher nur schwer möglich. Das wird umso problematischer, je länger der aufzuwickelnde Schlauch ist, wobei ein Schlauch mit großer Länge wegen der dann über einen längeren Zeitraum bzw. Weg einzubringenden höheren Heizenergie angestrebt wird, weil der Durchmesser des Wickelkörpers und/oder dessen Länge vergrößert werden müßten.

Bei im Markt befindlichen Schlauchwickelwärmern sind Durchmesser des zylindrischen Wickelkörpers und Nutlänge so ausgelegt, daß ein Schlauch mit 3,5 m Länge eingelegt werden kann, was es mit sich bringt, daß dieser mindestens neunmal um den Wickelkörper geführt werden muß. Das zum sorgfältigen Einlegen des Schlauches in die Nut notwendige punktgenaue Beobachten des Schlauchs bedingt somit, daß der Blickpunkt des Anwenders permanent in entsprechend großer Zahl ebenfalls um den Wickelkörper kreisen muß. Da diese Rotationsbewegung des Kopfes als ausgesprochen unangenehm empfunden wird, bleibt eine Sichtkontrolle häufig aus. Damit ist dann allerdings nicht sichergestellt, daß der Schlauch vollständig in die richtige Nut eingelegt ist, so daß es häufig zu in einem Nutgang übereinanderliegenden Schlauchwicklungen kommt. Ein falsch eingelegter Schlauch hat eine erheblich reduzierte Kontaktfläche zum Heizkörper, so daß die Wärmeübertragung und damit die Endtemperatur der Infusions- bzw. Transfusionsflüssigkeit verringert wird. Da am Venenzugang keine Messung der Flüssigkeitstemperatur erfolgt, bleibt dies zum Nachteil des Patienten unbemerkt.

Der Erfindung liegt daher die Aufgabe zugrunde, eine gattungsgemäße Vorrichtung ohne die genannten Nachteile zu schaffen, insbesondere so weiterzubilden, daß sich ein längerer Schlauch mit zudem weniger Aufwand und außerdem sicher aufwickeln läßt.

Diese Aufgabe wird erfindungsgemäß dadurch gelöst, daß der Wickelkörper von seiner Rückseite her mit einer schiefen, unter einem Verjüngungswinkel bezogen auf seine Mittellinie abfallenden Mantelfläche ausgebildet ist, wobei alle Nuten des Führungsgangs aus einer Blickposition einsehbar sind. Hiermit lassen sich mehrere Vorteile gleichzeitig erreichen. Der Anwender kann die gesamte Mantelfläche des z.B. kegelstumpfartigen oder auch pyramidenstumpfartigen bzw. gegebenenfalls eine ovale Grundfläche aufweisenden Wickelkörpers überblicken. Das Bedienungspersonal kann ohne eine lästige Rotationsbewegung des Kopfes bei gleichwohl stets freiem Blick auf die Mantelfläche den Schlauch einlegen und sich optimal auf dessen korrekte Positionierung in jedem Führungsgang bzw. jeder Nut konzentrieren. Die von vorne nach hinten schiefe bzw. abfallende Mantelfläche des Wickelkörpers unterstützt die Bewegung bzw. das Abgleiten des Schlauchs in die nächste freie Nut des Führungsgangs. Die Rotationsbewegung der Hand um den Wickelkörper verursacht nämlich eine gerichtete Kraft auf den Schlauch, so daß ein vom Anwender bzw. von der Bedienungsperson versehentlich weit oberhalb der freien Folgenut um den Wickelkörper gelegter Schlauchabschnitt selbsttätig in die richtige Position der freien Folgenut rutscht. Damit sind in einer Nut des Führungsgangs übereinanderliegende Schlauchlagen ausgeschlossen.

Da die gesamte Wickelkörper-Mantelfläche aufgrund der Verjüngung nach vorne hin im freien Blickfeld der Bedienungsperson liegt, kann weiterhin auch sofort festgestellt werden, ob mitunter nicht auszuschließende Lufteinschlüsse aufgetreten sind. Insgesamt ergibt sich somit, daß nicht nur eine erheblich verbesserte Bedienfreundlichkeit gegeben ist, da dem Anwender bzw. der Bedienungsperson eine nur noch geringe Aufmerksamkeit und Konzentration abgefordert wird, sondern auch die Sicherheit in der Anwendung erhöht wird.

Weitere Einzelheiten und Merkmale der Erfindung ergeben sich aus dem Patentanspruch und der nachfolgenden Beschreibung von in den Zeichnungen dargestellten Ausführungsbeispielen der Erfindung. Es zeigen:
- Fig. 1: eine Halbschnittdarstellung eines im Ausführungsbeispiel als Kegelstumpf ausgebildeten Wickelkörpers, bei der im oberen Teil die Seitenansicht auf die Oberfläche und im unteren Teil ein Querschnitt durch den Wickelkörper dargestellt ist;
- Fig. 2: den Wickelkörper nach Fig. 1 mit Blickrichtung einer Bedienungsperson dargestellt;
- Fig. 3: als Einzelheit des Wickelkörpers einen Querschnitt durch eine Nut des Führungsgangs mit eingelegtem Schlauch;
- Fig. 4: die Bewegungsrichtung eines Schlauchabschnitts, der unter Krafteinwirkung selbsttätig in die nächste freie Nut des Führungsgangs fällt;
- Fig. 5: eine Halbschnittdarstellung des Wickelkörpers mit einer aufgesetzten Wärmemanschette; und
- Fig. 6: als Einzelheit des Wickelkörpers nach Fig. 5 eine Nut des Führungsgangs mit eingelegtem Schlauch und aufgesetzter Wärmemanschette.

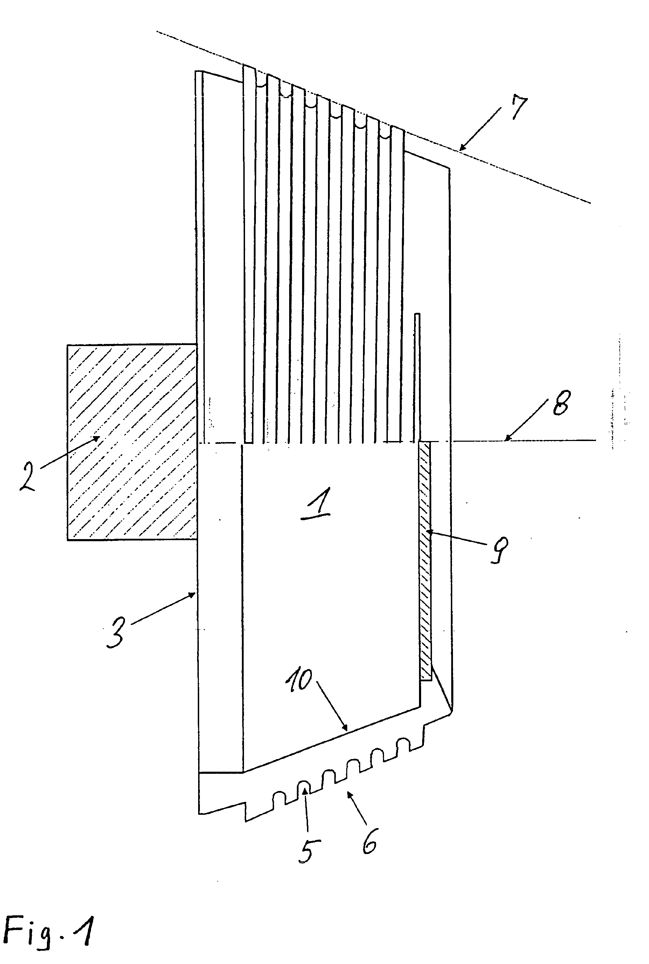

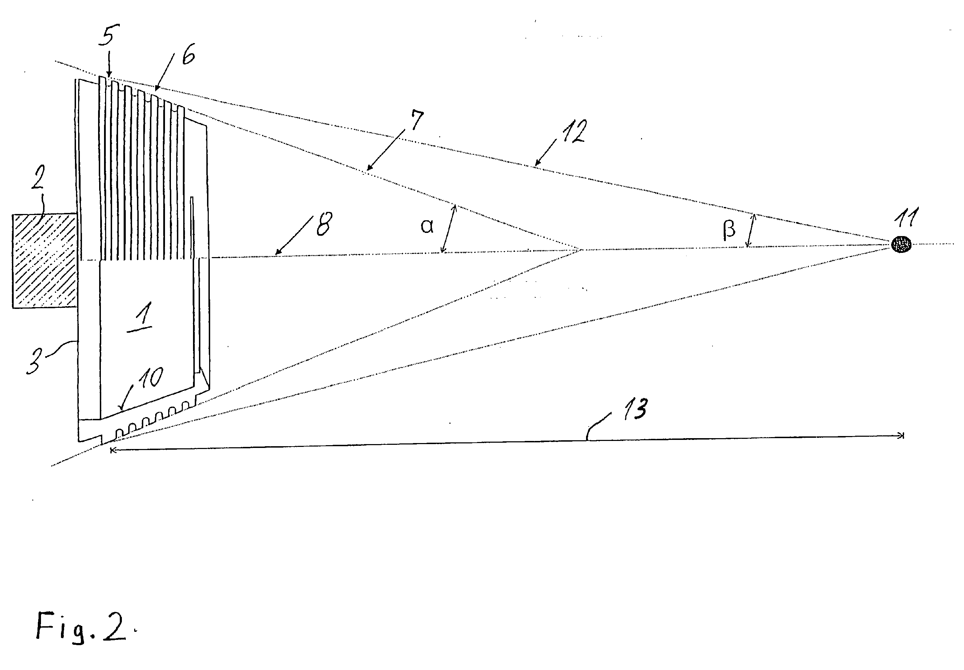

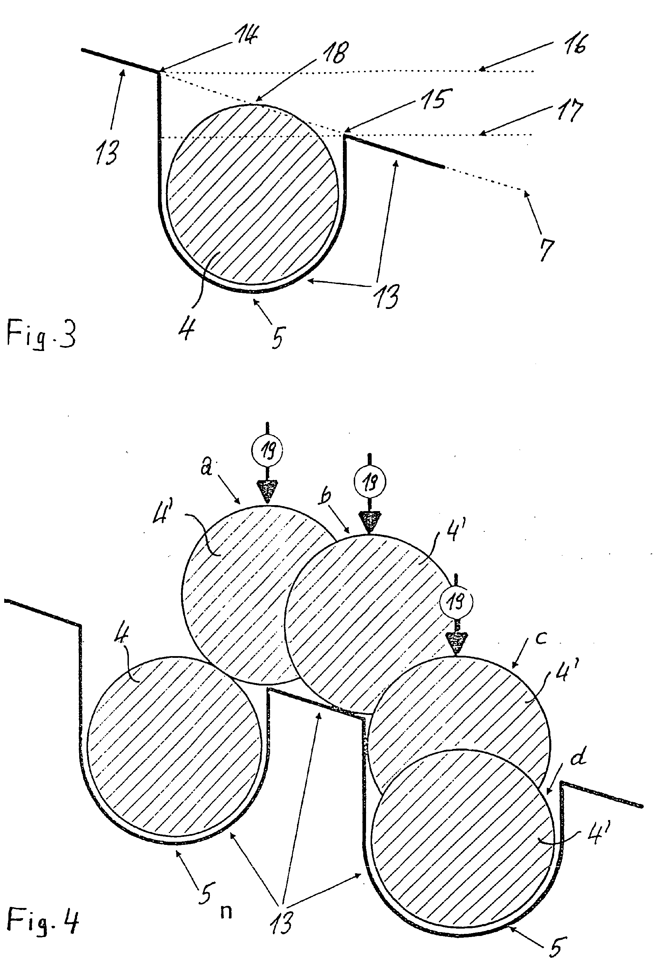

Ein Wickelkörper 1 nach den Fig. 1 sowie 2 und 5 ist zur Versorgung mit Heizenergie an eine Heizquelle angeschlossen (nicht dargestellt) und wird mit einer Klemmeinrichtung 2 an einen ebenfalls nicht gezeigten Infusionsständer in ca. 1,5 bis 2 m Höhe aufgehängt. Er ist von seiner Rückseite 3 her mit sich nach vorne hin stetig verringerndem Durchmesser ausgebildet und mit sich entlang seines Umfangs in seiner Mantelfläche erstreckenden Nuten 5 eines spiralartigen Führungsgangs 6 zur Aufnahme eines sukzessive in jede Nut 5 einzulegenden Schlauches 4 (vgl. die Fig. 3 und 4) versehen. Aufgrund der schiefen Ebene des Wickelkörpers 1 entsteht zwischen dessen Mantellinie 7 und seiner Mittellinie 8 ein Verjüngungswinkel α, wie in Fig. 2 gezeigt. Der zur Mittellinie 8 lotrecht gemessene Abstand zwischen der Mittellinie 8 und der Mantellinie 7 verringert sich folglich über den gesamten Umfang des Wickelkörpers 1 stetig nach vorne.

An der Vorderseite des Wickelkörpers 1 befindet sich eine Bedien- und Anzeigeeinheit 9 mit Bedientasten, über die eine Bedienperson die Temperaturwerte, auf die der Wickelkörper 1 temperiert werden soll, vorwählen kann. Zur Versorgung mit Heizenergie kann auf der inneren Mantelfläche 10 des hohlen Wickelkörpers 1 beispielsweise ein Folienheizelement aufgeklebt werden, das den Wickelkörper 1 definiert vorgewählt temperiert. Es wird somit die Funktion eines Wärmetauschers erfüllt, der eine aktiv erzeugte Wärme von innen über die Nuten 5 des Führungsgangs 6 auf den eingelegten Schlauch 4 überträgt.

Das für eine Bedienungsperson freie Blickfeld auf die schiefe, abfallende Mantelfläche des Wickelkörpers 1 wird anhand der Fig. 2 näher beschrieben. Eine Bedienungsperson kann aus ihrer Blickposition 11 die gesamte Mantelfläche überblicken, wobei der Blickwinkel β zwischen Blickrichtung 12 und Mittellinie 8 kleiner ist als der Verjüngungswinkel α. Ausgehend von einer durchschnittlichen Armlänge eines Menschen wird der Wickelkörper 1 so ausgelegt, daß der Abstand 13 zwischen der Blickposition 11 und der hinteren Nut 5 des Führungsgangs 6 ca. 50 cm beträgt. Bei einem Radius der Nuten 5 des Wickelkörpers 1 etwa zwischen 45 und 180 mm ergibt sich für den Winkel β ein Wertebereich von ca. 5 bis 20° und für den Verjüngungswinkel α eine untere Begrenzung von 5°; als Obergrenze kann aus konstruktiven Gründen ein Wert von 45° angenommen werden. Die Bedienungsperson kann ohne lästige Rotationsbewegung des Kopfes sowie mühelos den jeweiligen Schlauchabschnitt in die dazugehörige Nut 5 einlegen und sich optimal auf die korrekte Positionierung eines jeden Schlauchabschnitts konzentrieren, der stets frei im Blickfeld liegt.

Die Nuten 5 bzw. der Führungsgang 6 sind mit konstanter Tiefe in den Wickelkörper 1 eingebracht, wie Fig. 3 zu entnehmen ist. Die Formgebung der Mantelfläche wird durch eine Konturlinie 13 vorgegeben, wobei die Nut 5 durch einen oberen und einen unteren Nutpunkt 14 bzw. 15 begrenzt wird. Die Nutpunkte 14 bzw. 15 sind die Stellen auf der Konturlinie 13, an denen diese die Richtung der Mantellinie 7 verläßt und in die Nut 5 übergeht. Dieser Übergang kann eckig mit einer Kante (wie in Fig. 3), eckig mit mehreren Kanten, z.B. mit einer Fase, oder rund, z.B. mit einem Radius, gestaltet sein. Jede Nut 5 findet eine obere und eine untere Begrenzungslinie 16 bzw. 17 durch die in den Nutpunkt 14 bzw. 15 parallel verschobene Mittellinie 8. Die Tiefe der Nuten 5 des Führungsgangs 6 ist damit so bestimmt, daß der in eine Nut 5 eingelegte Schlauch 4 mit seiner höchsten Erhebung 18 in radialer Richtung weder die obere Begrenzungslinie 16 überschreitet noch die untere Begrenzungslinie 17 unterschreitet. Die genaue Lage des in die Nuten 5 des Führungsgangs 6 eingelegten Schlauchs 4 ist damit gewährleistet.

Wie in Fig. 4 verdeutlicht wird, bietet die schiefe Mantelfläche des Wickelkörpers 1 eine Einführhilfe für den Schlauch 4, da die Bewegung des einzulegenden Schlauchabschnitts in die nächste freie Nut unterstützt wird. Die Rotationsbewegung der Hand um den Wickelkörper 1 bewirkt eine gerichtete Kraft 19 auf den Schlauch 4, so daß ein Schlauchabschnitt 4', der vom Anwender versehentlich weit oberhalb der freien Nut n+1 um den Wickelkörper gelegt wird, wie durch die Position a verdeutlicht, von oberhalb der Nutposition n selbsttätig in die nächstfolgende freie Nut n+1 rutscht, wie durch die Einführsequenzen a bis d verdeutlicht.

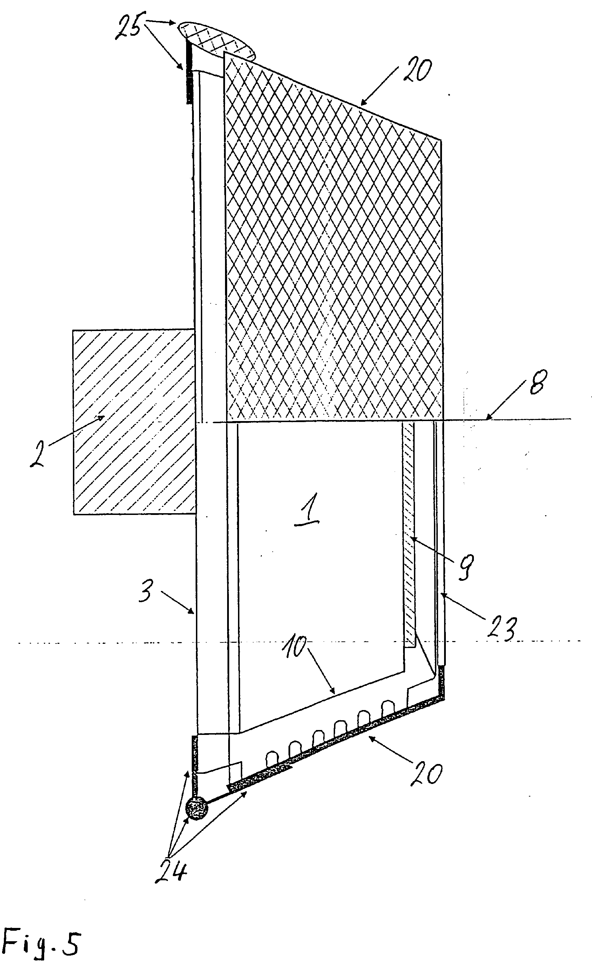

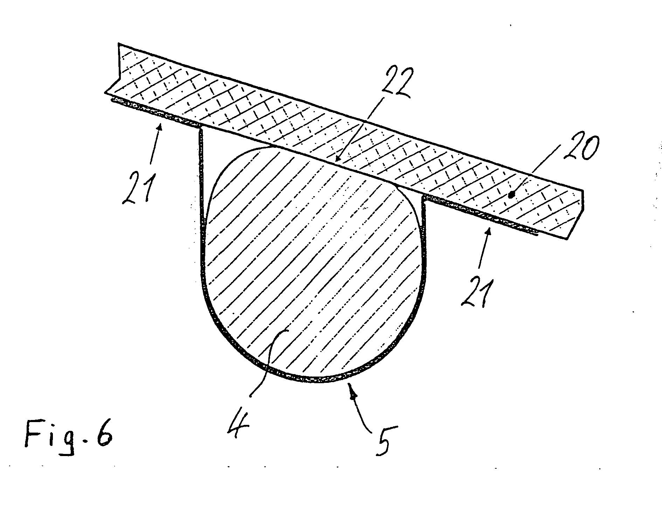

Der Wickelkörper 1 bietet gemäß Fig. 5 die Möglichkeit, eine einfach zu handhabende Wärmemanschette 20 aufzustülpen, der die gesamte Mantelfläche abdeckt und die keinen Kontakt zum Wickelkörper einnehmende Schlauchoberfläche zusätzlich erwärmt. Die Wärmemanschette 20 kann dabei als passives Heizelement genutzt werden, wenn sie gemäß Fig. 6 im angelegten Zustand Kontakt sowohl über die Flächen 21 zwischen benachbarten Nuten 5 des Führungsgangs 6 als auch zum Schlauch 4 über eine Fläche 22 hat.

Anders als bei einem zylindrischen Wickelkörper kann die Wärmemanschette 20 für den nach vorne abfallenden Wickelkörper 1 einteilig sein. Auf der Frontseite weist sie eine Öffnung 23 auf, die den Zugriff zur Bedien- und Anzeigeeinheit 9 erlaubt. Die Wärmemanschette 20 kann über ein Drehgelenk 24 mit der Rückseite bzw. -wand 3 des Wickelkörpers 1 mechanisch verbunden werden, so daß sie im geöffneten Zustand frei nach unten hängt. Zum Anlegen braucht sie dann lediglich nach oben geschwenkt zu werden und kann dort mit Hilfe eines Verschlußelements 25 am Wickelkörper 1 festgelegt werden. Das Verschlußelement 25 zieht die Wärmemanschette 20 in Richtung der Wickelkörper-Rückseite 3 und stellt somit sicher, daß sie sowohl die Mantelfläche als auch die Schlauchleitung berührt.

## Patentansprüche

1. Vorrichtung zum Temperieren von in einem Schlauch (4) geführter Flüssigkeit, insbesondere ein Blutprodukt oder eine Infusionslösung, umfassend einen Wickelkörper (1) mit sich entlang des Umfangs seiner Mantelfläche (10) erstreckenden, spiral- oder gewindeartigen, einen Führungsgang (6) zur Aufnahme des aufgewickelten Schlauches (4) bildenden Nuten (5),
**dadurch gekennzeichnet,**
**daß** der Wickelkörper (1) von seiner Rückseite (3) her mit einer schiefen, unter einem Verjüngungswinkel (α) bezogen auf seine Mittellinie (8) abfallenden Mantelfläche ausgebildet ist, wobei alle Nuten (5) des Führungsgangs (6) aus einer Blickposition (11) einsehbar sind.

## Claims

1. A device for maintaining the temperature of a liquid guided in a tube (4), in particular a blood product or an infusion solution, comprising a winding body (1) having spiral-shaped or thread-shaped grooves (5) extending along the circumference of the jacket surface (10) of the winding body and forming a guide (6) for receiving the wound tube (4),
**characterized in that**
from its back side (3), the winding body (1) is embodied with an inclined jacket surface sloping at a tapering angle (α) oriented towards its center line (8), whereby all of the grooves (5) of the guide (6) are visible from a viewing position (11).

## Revendications

1. Dispositif pour tempérer un liquide circulant dans un flexible (4), notamment un produit du sang ou une solution pour perfusion, comprenant une bobine, avec des rainures (5) hélicoïdales ou du type d'un filetage, s'étendant le long de la périphérie de sa surface d'enveloppe (10), formant un pas de guidage pour réceptionner le flexible (4) enroulé,
**caractérisé en ce que** la bobine (1) est formée à partir de sa face arrière (3) avec une surface d'enveloppe inclinée, déclinant sous un angle de conicité (α), par rapport à sa ligne médiane (8), toutes les rainures du pas de guidage (6) étant visibles à partir d'une position d'observation (11).
